(19) Europäisches Patentamt
European Patent Office
Office européen des brevets

(11) Veröffentlichungsnummer : **0 056 618**
**B1**

(12) # EUROPÄISCHE PATENTSCHRIFT

(45) Veröffentlichungstag der Patentschrift : **28.08.85**

(51) Int. Cl.⁴ : **C 07 K 1/08**, C 07 K 5/06, C 07 K 5/08, C 07 K 5/10

(21) Anmeldenummer : **82100218.5**

(22) Anmeldetag : **14.01.82**

(54) **Verfahren zur Herstellung von Carbonsäureamidgruppen enthaltenden Verbindungen, insbesondere von Peptiden.**

(30) Priorität : **17.01.81 DE 3101427**

(43) Veröffentlichungstag der Anmeldung : **28.07.82 Patentblatt 82/30**

(45) Bekanntmachung des Hinweises auf die Patenterteilung : **28.08.85 Patentblatt 85/35**

(84) Benannte Vertragsstaaten : **AT BE CH DE FR GB IT LI NL SE**

(56) Entgegenhaltungen :
**EP-A- 0 014 834**
**TETRAHEDRON LETTERS, no. 40, 1976 A.G. JACKSON et al: "Activation of Carboxylic Acids as Diphenylphosphinic mixed Anhydrides: Application to Peptide Chemistry" Seiten 3627-30**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Wissmann, Hans, Dr.**
**Falkenstrasse 12**
**D-6232 Bad Soden am Taunus (DE)**
Erfinder : **Kleiner, Hans-Jerg, Dr.**
**Altkönigstrasse 11a**
**D-6242 Kronberg/Taunus (DE)**

**EP 0 056 618 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Zur Herstellung von Carbonsäureamid- und Peptidbindungen ist eine große Anzahl von Verfahren bekannt (siehe z. B. Houben-Weyl, Methoden der organischen Chemie, Bd. XV, Teil II, S. 1-364. Ferner Angew. Chemie *92*, 129 (1980)). Alle diese Verfahren zielen mit unterschiedlichem Erfolg darauf, die für die Synthese von Peptiden notwendigen Kriterien der Racemisierungsfreiheit, der einfachen und schonenden Durchführbarkeit bei honen Austeuten und mit leicht zugänglichen, möglichst ungefährlichen Ausgangs-materialien sicherzustellen.

Aus der EP-A-14 834 ist ein Verfahren zur Herstellung von Carbonsäureamiden und Peptiden bekannt, das dadurch gekennzeichnet ist, daß man Verbindungen, die eine freie Aminogruppe enthalten, in Gegenwart von Anhydriden der Alkanphosphonsäuren mit Verbindungen umsetzt, die eine freie Carboxylgruppe enthalten.

Es wurde gefunden, daß man Carbonsäureamidgruppen enthaltende Verbindungen, insbesondere Oligopeptide, unter milden Bedingungen in guter Ausbeute herstellen Kann, indem man Verbindungen, die eine freie Aminogruppe enthalten, insbesondere Aminocarbonsäurederivate oder Peptide, deren Carboxylgruppe geschützt ist, in Gegenwart des Anhydrids einer Dialkylphosphinsäure mit Verbindungen umtsetzt, die eine freie Carboxylgruppe enthalten, insbesondere Aminocarbonsäuren oder Peptide, deren Aminogruppe acyliert ist.

Das erfindungsgemäße Verfahren stellt einen neuen Weg dar, die eingangs genannten Bedingungen für eine wirtschaftliche Synthese von Peptiden und Amiden zu optimieren.

Die Anhydridbindung in den bei dem Verfahren der EP-A-14 834 mutmaßlich als Zwischenprodukt auftretenden Pyrophosphonsäuren ist sehr reaktionsträge ; sie wird erst beim Kochen in wäßriger Lösung gespalten (s. Houben-Weyl Bd. XII/1, Seite 608, Abschnitt B), während die Phosphinsäureanhydride (s. Houben-Weyl Bd. VII/1, Seite 266, vorletzter Satz von Abschnitt g) mit Wasser in relativ kurzer Zeit zu den freien Phosphinsäuren hydrolysiert werden. Die aus der EP-A-14 834 bekannten Kondensationsmittel hydrolysieren ebenfalls sehr schnell mit Wasser unter Bildung der Oligo- und Pyrophosphonsäuren, so daß die Kupplung unter Ausschluß von Wasser erfolgen muß.

Es ist daher sehr überraschend und nicht aus dem Stand der Technik herleitbar, daß das erfindungsgemäße Verfahren nicht nur in wasserfreien, inerten Lösungsmitteln, sondern auch in Gemischen aus Wasser und einem mit Wasser mischbaren organischen Lösungsmittel durchgeführt werden kann, obgleich die Reaktion formal unter Abspaltung von Wasser verläuft und die Dialkylphosphinsäure-Anhydride bekanntlich relativ leicht hydrolysieren. Die Ausbeuten sind, unabhängig vom Reaktionsmedium, gut und der Racemisierungsgrad ist gering.

Die zum Schutz der funktionellen Gruppen eingeführten Reste Können im Falle der Synthese von Peptiden anschließend in üblicher Weise abgespalten werden.

Unter Anhydriden der Dialkylphosphinsäuren werden Verbindungen der Formel I verstanden

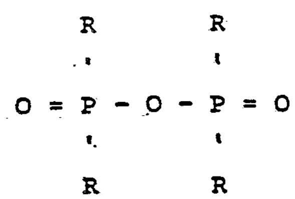

(I)

in der R Alkyl bedeutet. Die in der Formel gezeigten Substituenten R können gleich oder verschieden sein. Bevorzugt sind Anhydride, bei denen Beide P-Atome gleich substituiert sind.

Besonders geeignet im Sinne der Erfindung sind Anhydride der Formel I, in denen R jeweils ein niederes Alkyl ist, vorzugsweise ein solches mit 1 bis 4 C-Atomen.

Die erfindungsgemäß verwendeten Dialkylphosphinsäureanhydride sind farblose Flüssigkeiten. Sie sind bei Raumtemperatur stabil und lassen sich bei vermindertem Druck unzersetzt destillieren. In den meisten nicht wäßrigen Lösungsmitteln, insbesondere in Lipidlösungsmitteln wie Chloroform oder Methylenchlorid, aber auch in polaren Lösungsmitteln wie DMF und DMA sind sie leicht löslich.

Als Anhydride der Dialkylphosphinsäuren seien beispielsweise genannt : Methyläthylphosphinsäureanhydrid, Methylpropylphosphinsäureanhydrid, Methyl-butylphosphinsäureanhydrid, Diäthylphosphinsäureanhydrid, Di-n-propylphosphinsäureanhydrid, Di-n-butylphosphinsäureanhydrid.

Die Herstellung der Dialkylphosphinsäureanhydride kann in an sich bekannter Weise erfolgen, z. B. durch Umsetzung der Dialkylphosphinsäurechloride mit Dialkylphosphinsäurealkylestern bei 150-160 °C (Houben-Weyl, Methoden der Organischen Chemie, G. Thieme Verl., Stuttgart 1963, BD. XII, S. 266 ff). Besonders bevorzugt sind Verfahren, bei denen Dialkylphosphinsäure, deren Salze oder Ester mit Phosgen umgesetzt werden (DBP 2 129 583, DOS 2 225 545).

Die erfindungsgemäße Umsetzung wird vorzugsweise in neutralem oder schwach alkalischen Medium durchgeführt. Am einfachsten ist es, den pH-Wert des Mediums durch Zugabe von aliphatischen oder cykloaliphatischen tertiären Basen wie N-Methylmorpholin, N-Äthylmorpholin oder Trialkylaminen

mit bis zu 6 C-Atomen pro Alkylrest einzustellen. Beim Arbeiten in gemischtwässrigen Systemen können anstelle der organischen Base auch als Puffersysteme wirkende alkalische Salze z. B. solche der Kohlensäure und oder der Phosphorsäure verwendet werden.

Zur Herstellung von Oligopeptiden nach dem erfindungsgemäßen Verfahren verwendet man als Ausgangsmaterialien einerseits eine Aminosäure oder ein Peptid mit einer blockierten Carboxylgruppe und andererseits eine Aminosäure oder ein Peptid mit einer blockierten Aminogruppe.

Für den Schutz der Carboxylgruppen können alle in der Peptidsynthese üblichen Schutzgruppen verwendet werden. Insbesondere eignen sich Ester geradkettiger oder verzweigter aliphatischer Alkohole wie Methanol, Äthanol, tert. Butanol. Auch Ester araliphatischer Alkohole wie Benzylakohol oder Diphenylmethylcarbinol können Verwendung finden.

Zum Schutz der Aminogruppen können ebenfalls alle in der Peptidsynthese üblichen Schutzgruppen dienen. Als besonders geeignet seien beispielsweise der Carbobenzoxyrest und der Carbo-tert.-butyloxyrest genannt.

Als Lösungsmittel können alle in der Peptidsynthese üblichen wasserfreien, inerten Lösungsmittel, z. B. Methylenchlorid, Chloroform, Dimethylformamid, Dimethylacetamid, Dioxan oder Tetrahydrofuran Verwendung finden.

Die Synthese läßt sich auch in gemischtwässrigen Lösungsmittelsystemen durchführen. Hierunter werden Gemische aus Wasser und einem mit Wasser mischbaren organischen Lösungsmittel wie Dioxan/Wasser, Tetrahydrofuran/Wasser oder Dimethylformamid/Wasser verstanden. Die Verwendung solcher Systeme ist insbesondere bei der Verknüpfung von über wiegend wasserlöslichen Peptiden von Vorteil.

Die Reaktion läuft in der Regel bei Raumtemperatur hinreichend schnell ab. Leichtes Erwärmen schadet nicht. Höhere Temperaturen, etwa oberhalb 50 °C, sind, insbesondere bei der Peptidsynthese, wegen der Racemisierungsgefahr nicht zu empfehlen.

Die erfindungsgemäßen Dialkylphosphinsäureanhydride werden vorzugsweise im Überschuß (etwa 2-2,5 Mol Dialkylphosphinsäureanhydrid/Mol zu knüpfender Peptidbindung) eingesetzt. Sie können als solche unverdünnt dem Reaktionsgemisch zugetropft werden.

Das erfindungsgemäße Verfahren zeigt gegenüber den zur Zeit gebräuchlichen eine Reihe von Vorteilen.

Die Dialkylphosphinsäureanhydride liefern nach der Synthese keine schwerlöslichen Nebenprodukte, ein Vorteil der das erfindungsgemäße Verfahren vor der zur Zeit häufig verwendeten Peptidknüpfung unter Verwendung von Dicyclohexyl-carbodiimid auszeichnet.

Die Dialkylphosphinsäureanhydride sind leichter zugänglich und auch leichter zu handhaben als die meisten für diesen Zweck gebräuchlichen racemisierungsarmen Reagenzien.

Gegenüber den bisher beschriebenen Verfahren der Peptidsynthese unter Verwendung von Aktivierungsmitteln auf der Basis des 3- oder 5-wertigen Phosphors, beispielsweise den Peptidsynthesen nach der Phosphorazomethode (Liebigs Ann. Chem. 580, S. 68), den Synthesemethoden unter Verwendung von Diäthychlorphosphit oder Tetraäthylpyrophosphit (J. Am. Chem. Soc. *74,* 5304 (1952) und J. Am. Chem. Soc. *74,* 5307 und 5309) (1952)) und der Synthesemethode unter Verwendung von Polyphosphorsäureestern (Ber. *91,* (1958) S. 1073-1082) hat das erfindungsgemäße Verfahren den Vorteil der geringeren Racemisierung.

Das erfindungsgemäße Verfahren ist einfach durchzuführen und liefert Peptide in hoher optischer Reinheit und Ausbeute. Außerdem ist es sparsam und umweltfreundlich.

Die dialkylphosphinsäureanhydride sind niedrigmolekular, leicht zu erhalten und zu reinigen und zeigen einen hohen Anteil an reaktiven Gruppen pro Gewichtseinheit sowie eine gute Lipophilität. Die Dialkylphosphinsäureanhydride und die zugehörigen Dialkylphosphinsäuren sind lipidlöslich. Das ermöglicht eine Aufarbeitung wasserlöslicher Peptidderivate über einen ersten Fällungsschritt mit geeigneten Lipoidlösungsmitteln.

Die aus dem Dialkylphosphinsäureanhydrid im Verlauf der Peptidsynthese erhaltene Dialkylphosphinsäure kann aus den Restlösungen der Synthesereaktion wiedergewonnen werden. Eine Wiedergewinnung der Dialkylphosphinsäuren aus einer größeren Menge von wässrigen Synthesemutterlaugen ist durch Extraktion mit Lösungsmitteln wie Chloroform und Isobutanol und anschließender destillativer Aufarbeitung möglich. Dabei ist es von besonderem technischen Interesse, daß die Dialkylphosphinsäuren im Vakuum ohne Zersetzung destillierbar sind. Die so gewonnen en wiederaufgearbeiteten Dialkylphosphinsäuren können sodann leicht nach dem Verfahren der DOS 2 225 545 in die entsprechenden Dialkylphosphinsäureanhydride übergeführt werden.

Bei der beschriebenen gemischtwässrigen Arbeitsweise kann die organische Base durch in wässriger Lösung alkalisch reagierende Salze der Kohlensäure und oder der Phosphorsäure ersetzt werden, was die oben beschriebene Wiederaufarbeitung der Dialkylphosphinsäuren nach der Synthese vereinfacht. In diesem Fall kann auf den Extraktionsschritt verzichtet werden. Die Dialkylphosphinsäure kann dann nach Freisetzung direkt aus der abgedampften Synthesemutterlauge abdestilliert werden.

Beispiel 1

Carbonbenzoxy-Glyzylglyzinäthylester :

Zu der Lösung von 10,5 g (0,05 Mol) Carbobenzoxy-glyzin gibt man unter Rühren und guter Kühlung nacheinander bei 0 °C 7,0 g (0,05 Mol) H-Gly-$OC_2H_5$ · HCl, 15 ml N-Äthylmorpholin und 20 g Methyläthylphosphinsäureanhydrid. Man läßt unter weiteren Rühren auf raumtemperatur erwärmen. Nach 16-stündigem Stehen bei Raumtemperatur destilliert man das Lösungsmittel bei Raumtemperatur im Vakuum ab, und nimmt den Rückstand in einem Gemisch aus 200 ml Essigester und 100 ml 5 %iger Kaliumbisulfatlösung auf. Die Essigesterlösung wird noch zweimal mit 100 ml gesättigter Natriumbicarbonatlösung gewaschen, über Natriumsulfat getrocknet und im Vakuum abgedampft.

Ausbeute : 12,0 g (81 % d. Th.) Z-Dipeptidester mit einem Schmelzpunkt von 80 °C.

## Beispiel 2

Z-Val-Tyr ($Bu^t$)-His-$OCH_3$

Zu einer Suspension von 11,5 g H-His-$OCH_3$ · HCl in 100 ml Dimethylformamid gibt man nacheinander bei 0 °C unter Rühren 30 ml N-Äthylporpholin, 21,5 g Z-Val-Tyr ($Bu^t$)-OH und 20 g Methyläthylphosphinsäureanhydrid.

Die nach Abklingen der exothermen Reaktion praktisch klare Reaktionslösung Läßt man über Nacht bei Raumtemperatur stehen. Dann dampft man die Lösung im Vakuum bei Raumtemperatur ab und versetzt den Rückstand mit einem Gemisch aus 100 ml gesättigter $NaHCO_3$-Lösung und 200 ml Essigsäureäthylester. Das Rohprodukt wird unter Schütteln in die Essigesterphase überführt. Diese wird mit wenig Wasser gewaschen, über Natriumsulfat getrocknet und im Vakuum zur trockene gebracht. Der in Rückstand verbliebene Z-Tripeptidester erstarrt beim digerieren mit Diäthyläther.

Ausbeute : 21 g vom Schmelzpunkt 188-190 °C $[\alpha]_D = 8,2°$ (c = 1, DMF).

Aus der Mutterlauge können durch Abdampfen des Lösungsmittels und Umfällen aus Essigsäureäthylester/Diäthyläther weitere 2,5 g des Peptids gewonnen werden.

Gesamtausbeute : 76 % d. Th.

## Beispiel 3

Z-Pro-Ala-Lys(Boc)-Phe-$NH_2$

19,1 g H Lys(Boc)-Phe-$NH_2$ · HCl(0,044 Mol) löst man in 100 ml Dimethylformamid, gibt bei 0 °C unter Rühren 26 ml N-Athylmorpholin, 16,0 g (0,005 Mol) Z-Pro-Ala-OH und 17,5 g Methyläthylphosphinsäureanhydrid zu. Man läßt 48 Stunden bei Raumtemperatur stehen, dann bringt man die Reaktionsmischung im Vakuum zur Trockene, digeriert den Rückstand mit 100 ml 2n-Sodalösung, 100 ml 10 %iger wässriger Zitronensäurelösung und 100 ml destilliertem Wasser und trocknet im Vakuum über Phosphorpentoxyd.

Ausbeute : 30,4 g = 89 d. Th.

Schmelzpunkt : 163 °C $[\alpha]_D = 27°$ (C = 1, DMF)

## Beispiel 4

Z-ASP ($OBu^t$)-Phe-$NH_2$

1.6 g (0,01 Mol) H-Phe-$NH_3$ löst man zusammen mit 28 ml N-Äthylmorpholin bei 0 °C in 20 ml Dimethylformamid. Dieser Lösung setzt man unter Kühlung und Rühren 3,23 g (0,01 Mol) Z-ASP ($OBu^t$)OH und Methyläthylphosphinsäureanhydrid zu. Die so hergestellte Reaktionslösung Läßtman über Nacht bei Raumtemperatur stehen und arbeitet sie dann wie üblich durch Extraktion in Essigester, Waschen mit Wasser, Wässriger Natriumcarbonatlösung 5 % wässriger $KHSO_4$-Lösung, Einengen der über Natriumsulfat getrockneten Essigesterlösung und Fällen des Endproduktes mit Diäthyläther auf.

Ausbeute : 4,0 g (85 % d. Th.)

Schmelzpunkt : $[\alpha]_D = -33,1°$ (c = 1, $CH_3OH$)

## Beispiel 5

Z-Gly-Thr($Bu^t$)-Phe-$OCH_3$

In 120 ml Dimethylsulfoxyd (p-A. Merck) löst man nacheinander 18,6 g (0,05 Mol) H-Thr($Bu^t$)-Phe-$OCH_3$ · HCl, 30 ml (0,238 Mol) N-Äthylmorpholin und 10,4 g (0,05 Mol) Z-Gly-OH. Dann gibt man unter Eiskühlung und Feuchtigkeitsausschluß 4 g Methyläthylphosphinsäureanhydrid portionsweise unter Rühren zu. Man rührt noch 24 Studen bei Rautemperatur und gießt dann die Reaktionslösung in 500 ml gesättigte Natriumcarbonatlösung ein. Das Reaktionsprodukt fällt dabei aus. Man dekantiert von der überstehenden Lösung und nimmt die Fällung in Essigsäureäthylester auf. Die Essigesterlösung wäscht man mit Wasser, trocknet über Magnesium-sulfat, engt im Vakuum weitgehend ein und fällt das endprodukt mit Petroläther. Es kristallisiert über Nacht bei + 4 °C.

Ausbeute : 18,0 g (75 % d. Th.)
Schmelzpunkt : 95 °C $[\alpha]_D$ = + 26° (c = 1, DMF)

Beispiel 6

Z-Phe-cyclohexylamid

Man löst 3 g (0,01 Mol) Z-Phe-OH, 1,0 g (0,01 Mol ; 1,2 ml) Cyclohexylamin und 5 ml N-Äthylmorpholin in 30 ml DMF und gibt unter Eiskühlung und Rühren 4 g Methyläthylphosphinsäureanhydrid zu. Nach Abklingen der exothermen Reaktion (Temperaturanstieg bis + 10 °C) läßt man die Lösung unter Rühren auf Raumtemperatur erwärmen und arbeitet dann das Reaktionsgemisch wie unter Beispiel 1 beschrieben auf.
Ausbeute : 3,3 g (85 % d. Th.)
Schmelzpunkt : 167 °C $[\alpha]_D$ = −2,8° (c = 1, DMF).

Beispiel 7

Z-Phe-Arg-Trp-Gly-$OCH_3$

Man löst 2,26 g (0,005 Mol) Z-Phe-Arg-OH und 1,55 g (0,005 mol) H-Trp-Gly-$OCH_3$ · HCl unter Zugabe von 5 ml N-Äthylmorpholin bei 0 °C in 10 ml Dimethylformamid. Dann tropft man unter Rühren bei dieser Temperatur 2 g Methyl-äthylphosphinsäureanhydrid ein. Man rührt noch eine Stunde bei Raumtemperatur und läßt dann 20 Stunden bei Raumtemperatur stehen. Nach Abdestillieren des Dimethylformamids im Vakuum bei Raumtemperatur digeriert man den Rückstand mit 30 ml gesättigter Natriumcarbonatlösung und trocknet den so erhaltenen Festkörper im Vakuum über $P_2O_5$.
Ausbeute : 3,0 g (90 % d. Th.)
$[\alpha]_D$ = − 28° (C = 1, DMF)

Beispiel 8

H-Phe-ARG-Trp-Gly-$OCH_3$ · 2HCl

Man löst 3 g des Carbobenzoxy-tetrapeptids in 200 ml Methanol, verdrängt die Luft im Reaktionsgefäß durch Stickstoff und gibt dann 1 g 10 % Pd/Bariumsulfatkatalysator zu. Unter Rühren und Durchleiten von Wasserstoff hydriert man wie üblich. Der pH-Wert der Reaktionslösung, ermittelt durch elektrometrischer Messung, wird durch Zugabe von 1n-methanolischer Salzsäurelösung bei 4,0 gehalten. Die hydrierende Abspaltung des Carbobenzoxyrestes ist nach 2 1/2 Studen beendet, wie am Verbrauch an 1n-methanolischer HCl-Lösung und am Aufhören der $CO_2$-Entwicklung festgestellt werden kann. Man verdrängt nun den Wasserstoff in Reaktionsgefäß durch Stickstoff, saugt unter stickstoff vom Katalysator ab, bringt die Lösung im Vakuum bei Raumtemperatur zur Trockene und digeriert den Rückstand mit absolutem Diäthyläther.
Ausbeute : nach Trocknen im Hochvakuum : 2,5 g (praktisch quantitativ)
$[\alpha]_D$ = + 5,5° (c = 1, Eisessig)

Beispiel 8a

Bestimmung des Racemisierungsgrades mit Hochdruck-Flüssigkeitschromatographie :

Auf die Trennsäule eines Flüssigkeitschromatographen (Säulenmaße : 0,4 × 25 cm, Säulenfüllung Spherosil[R] XOA 600 Normatom[R] 5 μm) die mit dem Lösungsmittelgemisch Chloroform/Methanol/Wasser/Ameisensäure/Ammoniumformiat (900 : 400 : 30 : 7 : 2,5) äquilibriert wurde, trägt man 25 μl einer 0,4 %igen Lösung des Peptidderivates aus Beispiel 8 auf und entwickelt dann das Säulenchromatogramm durch durchpumpen des obengenannten Lösungsmittels durch die Säule mit einer Durchflßgeschwindigkeit von 2 ml pro Minute bei einem Druck von 141 bar. Die auf der Säule getrennten diastereomeren Peptide werden im Durchflußphotometer anhand ihrer Absorption bei 280 nm quantitativ bestimmt. Das D-Arg-Diastereomere des oben hergestellten Tetrapeptidesterhydrochlorids wurde unter den genannten Bedingungen etwa 10 Minuten nach dem Start der Säulentrennung eluiert, das L-Diastereomere erschien nach etwa 15 Minuten. Die Menge des D-Arg-Diastereomeren betrug 2 % des aufgetragenen Tetrapeptidesterhydrochlorids.

Beispiel 9

Z-Trp-Gly-$OCH_3$

Zu der Mischung von 3,35 g (0,05 Mol) Z-Trp-OH und 1,25 g H-Gly-Ome · HCl in 15 ml Dioxan/Wasser 1 : 1 gibt man bei 0 °C unter Rühren 6,5 ml N-Athylmorpholin. In die entstandene Lösung tropft man bei

der angegebenen Temperatur unter Rühren 4 g Methyläthylphosphinsäureanhydrid. Man läßt 10 Stunden bei Raumtemperatur stehen und destilliert dann die Lösungsmittel im Vakuum bei Raumtemperatur ab. Den Rückstand nimmt man in einem Gemisch aus 25 ml Wasser und 70 ml Essigester auf, trennt den Essigesterextrakt ab und Extrahiert die wäßrige Lösung noch Zweimal mit je 10 ml Essigester. Anschließend wäscht man die vereinigten Essigesterextrakte je zweimal mit 7 ml Wasser, 5 %iger Kaliumsulfatlösung (bis zur sauren Reaktion) und mit gesättigter $NaHCO_3$-Lösung bis zur schwach alkalischen Reaktion. Nach Trocknen über Magnesiumsulfat und Klären mit Aktivkohle destilliert man das Lösungsmittel im Vakuum weitgehend ab und digeriert den Rückstand mit Diisopropyläther.

Ausbeute : nach Trocknen im Vakuum über Phosphorpentoxyd 2,86 g = 70 % d. Th. farblose Kristalle Schmelzpunkt 157 °C $[\alpha]_D = -12,5°$ (c = 1, Eisessig)

## Beispiel 10

Z-Trp-Gly-$OCH_3$

Zu der Suspension von 3,35 g (0,01 Mol) Z-Trp-OH und 1,25 g (0,01 Mol) H-GLY-$OCH_3$, in 15 ml Dimethylformamid gibt man bei 0 °C portionsweise insgesamt 7 ml Wasser und anschließend 5,1 g feingepulvertes Natriumbicarbonat. Die Suspension klärt sich weitgehend nach Zutropfen von 4 ml Methyläthylphosphinsäureanhydrid. Eine mit Wasser verdünnte Probe der Reaktionslösung reagiert neutral. Nach Aufwärmen auf Raumtemperatur rührt man über Nacht nach und destilliert dann die Lösungsmittel im Hochvakuum bei Raumtemperatur ab. Den Rückstand nimmt man in einem Gemisch aus 25 ml Wasser und 70 ml Essigester auf, trennt den Essigesterextrakt ab, und extrahiert die Wässrige Phase noch zweimal mit je 10 ml Essigester. Anschließend wäscht man die vereinigten Essigesterextrakte je zweimal mit 7 ml Wasser, 5 %iger Kaliumbisulfatlösung (bis zur sauren Reaktion) und mit gesättigter Natriumbicarbonatlösung (bis zur schwach alkalischen Reaktion). Nach Trocknen über Magnesium-sulfat destilliert man die Essigesterterlösung im Vakuum weitgehend ab, und digeriert den Rückstand mit Diisopropyläther.

Ausbeute nach Trocknen i. V. über $P_2O_5$ : 3,0 g (73 % d. Th) fp : 157 °C $[\alpha]_D = -12,9°$ (c = 1, Eisessig)

## Beispiel 11

Z-Trp-Gly-$OCH_3$

Zu einer Mischung aus 3,35 g (0,01 Mol) Z-Trp-OH, 1,25 g (0,01 mol) H-Gly-$OCH_3 \cdot HCl$ und 15 ml Dimethylformamid gibt man unter Rühren bei 0 °C eine Lösung von 7,0 g $K_3PO_4 \cdot H_2O$ in 8 ml Wasser. Dann Tropft man unter weiterem Rühren 4,0 ml Methyläthylphosphinsäureanhydrid zu, und läßt anschließend unter Rühren auf Raumtemperatur erwärmen. Nach 24 Stunden Rühren bei Raumtemperatur destilliert man die Lösungsmittel im Vakuum bei Raumtemperatur ab, nimmt den Rückstand in einem Gemisch aus 25 ml Wasser und 80 ml Essigsäureäthylester auf, extrahiert die verbleibende Wasserphase ein Zweites Mal mit 20 ml Essigester und schüttelt die vereinigten Essigesterextrakte je dreimal mit 8 ml 5 %iger Kaliumbisulfatlösung und gesättigter Natriumbicarbonatlösung. Nach Trocknen der Essigesterlösung mit Natriumsulfat isoliert man das Produkt durch Abdampfen des Lösungsmittels und Digerieren des Rückstandes mit niedrigsiedendem Petroläther (Kp 40-80 °C)

Ausbeute nach Trocknen im Hochvakuum über $P_2O_5$ : 3,0 g (73 % d. Th.) Fp : 157 °C $[\alpha]_D = -12,9°$ (c = 2, Eisessig)

**Patentansprüche**

1. Verfahren zur Herstellung von Carbonsäureamidgruppen enthaltenden Verbindungen, insbesondere Peptiden, dadurch gekennzeichnet, daß man Verbindungen, die eine Carboxylgruppe enthalten, in Gegenwart von Dialkylphosphinsäureanhydriden mit Verbindungen, die eine freie Aminogruppe enthalten umsetzt und gegebenenfalls zum Schutz anderer funktioneller Gruppen eingeführte Reste abspaltet.

2. Verfahren nach Anspruch 1, dadurch gekennzeichnet, daß die Reaktion in gemischtwässrigem Medium durchgeführt wird.

3. Verfahren nach Anspruch 2, dadurch gekennzeichnet, daß das Reaktionsmedium mit Alkalisalzen der Kohlensäure und/oder der Phosphorsäure gepuffert wird.

**Claims**

1. A process for the preparation of compounds containing carboxylic acid amide groups, in particular peptides, which comprises reacting compounds containing a carboxy group, in the presence of dialkylphosphinic acid anhydrides, with compounds containing a free amino group and splitting off radicals that may have been introduced to protect other functional groups.

2. The process of claim 1, which comprises carrying out the reaction in a mixed aqueous medium.

3. The process of claim 2, which comprises buffering the reaction medium with alkali metal salts of carbonic acid and/or phosphoric acid.

**Revendications**

1. Procédé de préparation de composés contenant des groupes carboxyamide, en particulier des peptides, caractérisé en ce qu'on fait réagir des composés qui contiennent un groupe carboxyle, en présence d'anhydride d'acides dialcoyl-phosphiniques, avec des composés qui contiennent un groupe amino libre et éventuellement, on élimine les radicaux introduits pour la protection d'autres groupes fonctionnels.

2. Procédé selon la revendication 1, caractérisé en ce que la réaction est effectuée dans un milieu mixte aqueux.

3. Procédé selon la revendication 2, caractérisé en ce que le milieu de réaction est tamponné avec des sels alcalins de l'acide carbonique et/ou de l'acide phosphorique.